# EUROPÄISCHE PATENTSCHRIFT

(11) **EP 3 917 373 B1**
(45) Veröffentlichungstag und Bekanntmachung des Hinweises auf die Patenterteilung: **12.06.2024**
(21) Anmeldenummer: 20704210.2
(22) Anmeldetag: 30.01.2020
(51) Int. Cl.: A61B 1/005, A61B 1/00, A61B 1/31, A61B 1/018

(54) **ENDOSKOP MIT DISTALEM SCHWENKMECHANISMUS UND FEINJUSTIERUNG**
ENDOSCOPE HAVING DISTAL PIVOT MECHANISM AND FINE ADJUSTMENT
ENDOSCOPE COMPRENANT UN MÉCANISME PIVOTANT DISTAL ET UN AJUSTEMENT FIN

(30) Priorität: 01.02.2019 DE 102019102599
(43) Veröffentlichungstag der Anmeldung: 08.12.2021
(73) Patentinhaber: Bob, Konstantin, 69469 Weinheim (DE)
(72) Erfinder: Bob, Konstantin, 69469 Weinheim (DE)
(74) Vertreter: Winter, Brandl - Partnerschaft mbB
(86) Internationale Anmeldenummer: PCT/EP2020/052351
(87) Internationale Veröffentlichungsnummer: WO 2020/157233

(56) Entgegenhaltungen:
- EP-A1- 2 581 031
- DE-A1-102018 110 620
- US-A1- 2005 090 809
- US-A1- 2008 287 741

## Beschreibung

### Technisches Gebiet

Die vorliegende Erfindung betrifft ein Endoskop, insbesondere ein kombiniertes Kolos-/Duodenoskop mit einem Endoskopkopf, der vorzugsweise an einem, aktiv abkrümmbaren, distalen Endabschnitt eines Endoskopschaftes angeordnet ist und der zumindest eine Optik zur Bildgebung aufweist und einem Arbeitskanal, der sich in oder an dem Endoskop entlang dessen Längsrichtung erstreckt und dessen distaler Arbeitskanalausgang am oder im Endoskopkopf angeordnet ist, wobei der Endoskopkopf einen Schwenkmechanismus hat bzw. dem Endoskopkopf ein Schwenkmechanismus zugeordnet, dessen Schwenkradius unterschiedlich (kleiner) zum Krümmungsradius des ggf. vorhandenen aktiv abkrümmbaren distalen Endabschnitt (nachfolgend als Deflecting bezeichnet) ist..

### Hintergrund der Erfindung

Endoskope sind medizinische Arbeitsgeräte zur visuellen Exploration von Hohlräumen in einem Patientenkörper. Sie weisen grundsätzlich optische Einrichtungen am distalen, d.h. vom Anwender abgewandte Endoskopende (auch Endoskopkopf genannt) sowie optional einen Arbeitskanal auf, der sich ausgehend von einem proximalen (anwenderzugewandten) Endoskopabschnitt oder extrakorporalen Endoskopgriff durch einen (daran sich anschließenden) flexiblen/biegesteifen oder starren Endoskopschaft hindurch bis zum Endoskopkopf ersteckt und das extrakorporale Einführen und Verwenden eines medizinischen Instruments wie z.B. einer Zange, Schere, Nadel, Schlinge, Messer und dergleichen ermöglicht.

Derartige Endoskope können wahlweise mit zusätzlichen Funktionen versehen werden, etwa indem am distalen Endoskopende/Endoskopkopf eine Kappe oder Hülse auf den Endoskopkopf radial außenseitig aufgesetzt wird, die mit bestimmten Funktionen/Funktionselementen versehen oder ausgerüstet ist, wodurch das Endoskop nicht nur zur Exploration und/oder als Zugang für therapeutische Anwendungen sondern auch selbst als ein minimalinvasives Instrument zur Ausführung eines chirurgischen Vorgangs genutzt werden kann. Alternativ hierzu ist es aber auch vorgesehen, spezielle Endoskope für ganz bestimmte medizinische Anwendungen mit derartigen Fähigkeiten integral auszustatten, wobei derartige Spezialanfertigungen dann nur für diese besondere Anwendung geeignet sind.

Verschiedene diagnostische und/oder therapeutische Verfahren erfordern beispielsweise eine Bildgebung und/oder bei Bedarf therapeutische Techniken am Gallen- und/oder Bauchspeicheldrüsengang sowie den Lebergängen des Patienten. Da die Vatersche Papille, welche den gemeinsamen Austritt von Gallen- und Bauchspeicheldrüsengang in das Duodenum bildet, seitlich in das Duodenum hineinragt, sind herkömmliche prograde (in Endoskoplängsrichtung blickende) Endoskope für derartige Eingriffe ungeeignet, da nicht genügend Schwenkraum im engen Duodenum (Durchmesser 3 bis 4 cm) vorhanden ist, um deren prograde Optik sowie den Arbeitskanal in eine seitwärts blickende Position auszurichten, da ein typischer Biegedurchmesser solcher Geräte bei etwa 12 cm liegt.

### Stand der Technik

Aus dem Stand der Technik (bspw. der US 2010/228086 A) sind zu diesem Zweck speziell angefertigte Duodenoskope bekannt, welche eine laterale (seitwärts blickende) und retrograde (rückblickende) Optik (auch "Seitoptik" genannt) sowie einen ggf. seitwärts gerichteten/sich öffnenden Arbeitskanal aufweisen. Am Ausgang eines in Schaftlängsrichtung sich öffnenden Arbeitskanals solcher Duodenoskope ist in der Regel ein sogenannter Albarranhebel vorgesehen, der durch Verschwenken ein gezieltes Führen/Umlenken eines im Arbeitskanal geführten Werkzeugs zur Seite hin (in Radialrichtung des Endoskopschafts) ermöglicht. Durch die seitwärts gerichtete Anordnung der Funktionseinheiten am Endoskopkopf wird eine Bildgebung und Behandlung im Bereich des Duodenums unter optimaler Nutzung des zur Verfügung stehenden Raums im Patientendarm ermöglicht. Die Schriften US 2005/090809 A1 und DE 10 2018 110620 A1, die als nächstliegenden Stand der Technik betrachtet werden, beschreiben Endoskope mit schwenkbarem distalen Ende, wobei der Schwenkmechanismus keilförmig ausgebildetet Segmente aufweist.

Derartige Endoskope mit Seitoptik sind jedoch sehr aufwendig und teuer in ihrer Fertigung und werden deshalb bislang als wiederverwendbare Geräte entwickelt und hergestellt. Der gekrümmte Arbeitskanal solcher Endoskope sowie die komplexe und hinterschnittreiche Konstruktion des Albarranhebels haben sich in der Praxis zwar als sterilisierbar erwiesen aber der Sterilisationsprozess hat sich als zu materialermüdend für die empfindlichen Geräte herausgestellt. Deshalb werden die Geräte in der Praxis nach einem Eingriff lediglich desinfiziert. Dies hat zur Folge, dass nach einem Eingriff ein Bakterienrasen (Biofilm) im Arbeitskanal und/oder dem Hilfskanal des Endoskops bestehen bleiben kann. Löst sich dieser Biofilm dann bei einem darauf folgenden Eingriff, etwa weil ein Instrument durch den Arbeitskanal geschoben wird, so kann er bspw. in den Gallengang und/oder Pankreasgang gelangen und dort schwerwiegende Entzündungen bis hin zu einer Sepsis beim Patienten verursachen.

Weiter haben solche Geräte den Nachteil, dass die Navigation im Körper mit seitwärts blickenden Endoskopen im Allgemeinen eher schwierig ist, da weder die Optik noch der Arbeitskanal in dem engen Duodenum in prograde Richtung gerichtet werden können. Um ein Vorausblicken mit solchen Endoskopen zu bewerkstelligen ist ein Abkrümmen des dem Endoskopkopf unmittelbar vorgeordneten "Deflectings" (aktiv abkrümmbarer Endoskopschaft-Abschnitt) um ca. 90° erforderlich, wodurch wiederum mehr Platz in Endoskopquerrichtung benötigt wird, der nur im Magen vorhanden ist. Bei einem Vorausblicken mit 90° Abkrümmung des Deflectings im Duodenum oder anderen vergleichsweise engen Abschnitten des Magen-Darm-Traktes bestünde die Gefahr einer Verletzung der Schleimhaut des Lumens oder gar einer Darmperforation. Deshalb können solche Seitblick-Endoskope nur für wenige, sehr spezifische Eingriffe im Bereich des Duodenums eingesetzt werden.

Das im distalen Abschnitt des Schafts angeordnete Deflecting eines prograden (geradeausblickenden) flexiblen Endoskops mit über das Deflecting abwinkelbarer Spitze besteht zumeist aus gelenkig verbundenen Ringelementen, welche die Stützstruktur des Schafts bilden und über Bowdenzüge, häufig Biegesteuerzüge genannt, bedient und gegeneinander gekippt werden. Um das Einführen in den Hohlraum zu erleichtern und das Eindringen von Substanzen zu verhindern, sind die Ringelemente von einer flexiblen Hülle aus einem Kunststoffmaterial umgeben. Im Inneren der Ringelemente verlaufen insbesondere Licht- und Bildleitkabel, Kanäle für Fluide oder endoskopische Arbeitsinstrumente. Die Biegesteuerzüge sind entlang der Außen- oder Innenseite der Ringelemente geführt. Derartige flexible Endoskope sind beispielsweise in US 6,270,453 B1, US 6,482,149 B1 oder DE 101 43 966 B4 offenbart.

Dabei ist der kleinste Radius, den der biegsame bzw. gelenkige Abschnitt des Schafts einnehmen kann, durch das jeweilige Konstruktionsprinzip vorgegeben. Beispielsweise erlaubt ein mit aufeinander folgenden, jeweils gelenkig miteinander verbundenen Ringelementen ausgebildeter Gelenkabschnitt nur einen relativ großen Biegeradius, da die einzelnen Ringelemente jeweils nur einen geringen Kippwinkel zum nächsten Ringelement aufweisen. Wenn die gelenkige Verbindung zweier Elemente nur eine Kippung um eine Achse ermöglicht, ist es für eine räumliche Biegemöglichkeit erforderlich, Elemente mit abwechselnd gegeneinander verdrehten Kippachsen anzuordnen, so dass nur jedes zweite Element in eine jeweils gewünschte Richtung auslenkbar ist; hierdurch wird der mögliche minimale Biegeradius nochmals vergrößert. Der Nahbereich neben dem Schaftende ist daher mit einer in der Endoskopspitze angeordneten, vorausblickenden Optik nicht einzusehen, was im Dickdarmbereich, wo seitliche Ausstülpungen physiologisch vorkommen, dazu führt, dass Polypen oder Tumore bis zu einer Größe von etwa 1 cm übersehen werden können, weil sie seitlich in besagten Ausstülpungen liegen.

In diesem Zusammenhang sei darauf hingewiesen, dass ein Deflecting gemäß vorstehender Beschreibung dafür vorgesehen und ausgebildet ist,
- ein Einschieben des Endoskops beispielsweise in ein Patientenkolon zu erleichtern, indem das Defelcting aktiv an die Colon-Krümmungen angepasst werden kann und
- ein Heranführen des Endoskopfs an die Darmwand zu ermöglichen.

Demzufolge ist der Krümmungsradius eines solchen Deflecting vergleichsweise groß.

Aus dem Stand der Technik sind, wie bspw. in der DE 10 2013 222 279 A1 oder der DE 10 2012 220 578 A1 beschrieben, Endoskope mit einer separat zum restlichen Endoskopkopf schwenkbaren Optik bekannt, welche sowohl in prograde als auch in seitliche Richtung blicken können. Solche Endoskope weisen aber keinen Arbeitskanal auf (werden also rein diagnostisch verwendet) oder weisen einen festen Arbeitskanal in prograder Richtung auf und sind somit nicht für die typischen Einsatzzwecke von Duodenoskopen geeignet, die einen seitwärts/radial ausgerichteten Arbeitskanal oder eine Umlenkvorrichtung voraussetzen.

Zusammengefasst kann man sagen, dass der Anwender mit den aus dem Stand der Technik bislang bekannten Duodenoskopen nicht in der Lage ist, sowohl in prograde Richtung als auch im seitlichen / rückblickenden Nahbereich des Endoskops minimalinvasive Eingriffe vorzunehmen und Polypen oder Tumore zu erkennen (Dickdarmuntersuchung).

### Kurzbeschreibung der Erfindung

Angesichts der vorstehend beschriebenen Nachteile des Stands der Technik, ist es die Aufgabe der vorliegenden Erfindung, einen (gegenüber einem bekannten Deflecting) feinjustierbaren Schwenkmechanismus für einen Endoskopkopf mit vergleichsweise kleinem Schwenkradius bereitzustellen, der die Beobachtung und die integrierte minimalinvasive chirurgische Behandlung des die Endoskopspitze umgebenden Nahbereiches insbesondere im Rahmen einer Duodenoskopie ermöglicht.

Diese Aufgabe wird durch ein Endoskop (kombiniertes Gastro-/Duodenoskop) gemäß Anspruch 1 gelöst. Vorteilhafte Weiterbildungen sind Gegenstand der Unteransprüche.

Die Lösung der vorstehenden Aufgabe sieht prinzipiell das Vorsehen einer Mehrzahl technischer Maßnahmen vor, die in ihrer Kombination zu einer gegenüber bekannten Deflecting-Konstruktionen deutlich kleineren Schwenkradien führen. Im Konkreten hängt der minimal mögliche Schwenk-/Abkrümmradius des erfindungsgemäßen (feinjustierbaren) Schwenkmechanismus gemäß einem ersten Aspekt der Erfindung im Wesentlichen von der Form zumindest eines oder mehrerer zueinander verschwenk-/abwinkelbarer Segmente (des Endoskopkopfes) des Schwenkmechanismus sowie der Positionierung des Arbeitskanals innerhalb des jeweiligen Segments ab. Entscheidend dabei ist, dass die Segmentform in einem Abkrümmrichtungsbereich möglichst flach/dünn und in einem diametral gegenüberliegenden Abstützbereich möglichst dick baut, sodass zwei aneinander anliegende/angrenzende Segmente in die vorbestimmte Abkrümmrichtung um einen vergleichsweise großen Winkel zueinander verschwenken/abklappen können und dass diese Verschwenkung/Abklappung nicht oder möglichst wenig durch den Arbeitskanal des Endoskops beeinträchtigt wird. Letzteres wird dadurch erreicht, indem der Arbeitskanal nicht mehr zentrisch sondern außermittig in den dicken Bereich des jeweiligen Segments platziert ist.

In anderen Worten ausgedrückt ist gemäß dem ersten Aspekt der vorliegenden Erfindung zumindest ein Segment des erfindungsgemäßen (feinjustierbaren) Schwenkmechanismus, welches unmittelbar an den (eine Optik sowie einen Arbeitskanal-Ausgang aufweisenden) Endoskopkopf bzw. Teil des Endoskopkopfs angrenzt und damit quasi dem Endoskopkopf (als ein Bestandteil von diesem) zugeordnet werden kann, als eine in Draufsicht im Wesentlichen runde und in Seitenansicht im Wesentlichen keilförmige Scheibe/Platte ausgebildet. Demzufolge hat die/jede Scheibe/Platte in Seitenansicht betrachtet einen flachen Umfangsabschnitt der sich in Richtung hin zum diametral gegenüberliegenden Umfangsabschnitt kontinuierlich (keilförmig) verdickt und an diesem gegenüberliegenden Umfangsabschnitt sein Plattendicken-Maximum erhält. Dabei wird dieses Plattendickenmaximum über einen (kleinen) Teilumfangsabschnitt beibehalten, wodurch sich eine (gegenüber dem Keilformbereich kleine) Aufstandsfläche ergibt, die im Wesentlichen senkrecht zur Platten-Mittelachse ausgerichtet ist. In Draufsicht gesehen ist des Arbeitskanal dezentral zumindest teilweise in diesem Teilumfangsabschnitt mit maximaler Plattendicke ausgebildet.

In prograder Ausrichtung des Endoskopschafts bzw. des erfindungsgemäßen feinjustierbaren Schwenkmechanismus stützen sich der Endoskopkopf und das vorstehend beschriebene Plattensegment und/oder das vorstehend beschriebene Plattenelement und ein weiteres, daran angrenzendes Plattenelement des Schwenkmechanismus mit vorzugsweise gleichem Aufbau an den (kleinen) Teilumfangsabschnitten aneinander ab, wodurch diese prograde Ausrichtung stabil gehalten werden kann. Wird nunmehr ein Betätigungsmechanismus (z.B. ein Bowdenzug) aktiviert klappen der Endoskopkopf und das eine beschriebene Plattensegment und/oder das eine beschriebene Plattensegment und das weitere, daran angrenzende Plattensegment zueinander ab, wobei sich der flache Abschnitt des/der Segment(e) einander annähern und letztlich einander berühren.

Der maximale Abklapp-/Schwenkwinkel entspricht als dem aufgespannten Keilwinkel zwischen den sich jeweils gegenüberliegenden Segmenten, der durch den Arbeitskanal nicht/marginal durch den Arbeitskanal eingeschränkt wird, da sich dieser zumindest teilweise außerhalb des Keilformbereichs ausbildet.

Das erfindungsgemäße Endoskop weist demzufolge in einer bevorzugten Ausführungsform einen langgestreckten Grundkörper und an seinem distalen (vom Anwender abgewandten) Endabschnitt einen distal (in der Regel/vorzugsweise distal an eine aktiv abkrümmbare sogenannte "Deflectingzone") angeordneten Endoskopkopf bzw. eine distale Endkappe mit zumindest einer Optik zur Bildübertragung, einem Leuchtmittel und einen Arbeitskanal zur Führung von Werkzeugen und/oder zur Durchströmung von Medien auf. Zudem weist das erfindungsgemäße Endoskop den internen, zum allgemein bekannten "Deflecting" unterschiedlichen (d.h. falls vorhanden in Richtung distal davon beabstandeten zusätzlichen) Abklapp- oder Schwenkmechanismus auf, der dazu ausgebildet ist, zumindest einen Abschnitt des Endoskopkopfs von einer prograden Ausrichtung in eine laterale bzw. seitwärts oder retrograd gerichtete Ausrichtung, d.h. um eine Schwenkachse quer zur Endoskoplängsrichtung, insbesondere stufenlos ggf. aber auch gerastert, zu verschwenken. Zumindest die Optik und der Arbeitskanal sind direkt oder mittelbar mit dem Schwenkmechanismus gekoppelt, derart, dass sie gemeinsam mit dem Schwenkmechanismus bzw. dem von diesem verschwenkbaren Endoskopkopfabschnitt verschwenkt werden. In anderen Worten bildet der erfindungsgemäße Endoskopkopf eine verschwenkbare Optik- und Arbeitskanaleinheit aus, sodass ein mit diesem bestücktes/ausgebildetes Endoskop sowohl prograd als auch seitwärts / retrograd ausgerichtet einsetzbar ist. Das Wort "Optik" steht im Rahmen dieser Anmeldung zusammenfassend für alle dem Stand der Technik im Bereich der Endoskopie bekannte Bildgebungseinrichtungen, wie bspw. einem Modul aus CMOS- oder CCD-Chip mit Objektiv und Leuchtmittel (LED) im Endoskopkopf oder die Verwendung von Lichtleitern etc. Dabei ist es unerheblich, ob die Grundstellung des Schwenkmechanismus prograd oder lateral ist. Zur Umsetzung des Erfindungsgedankens kommt es lediglich darauf an, dass der Schwenkmechanismus beide Ausrichtungen einnehmen kann, sodass ein erfindungsgemäßes Endoskop die Funktionen eines prograden Endoskops und eines Endoskops mit Seitoptik in sich vereint.

Gemäß dem ersten Aspekt der Erfindung kann der Schwenkmechanismus eine Anzahl (oder Mehrzahl) axial aufeinanderfolgender und mittels zumindest eines Betätigungselements aktiv gegeneinander winkelverstellbare Segmente aufweisen, die in Axialrichtung zumindest einen (Arbeits-)Kanal zur Durchführung von minimalinvasiven chirurgischen Instrumenten, Spülmedien, Versorgungsleitungen und dergleichen definieren. Die Segmente können in diesem Fall als keilförmige Zylinderausschnitte (Platten) mit keilförmig zueinander ausgerichteten Stirnseiten ausgebildet sein, wodurch jeder Zylinderausschnitt einen Zylindermantelabschnitt mit minimaler Axiallänge (minimale Plattendicke) und einen gegenüberliegenden Zylindermantelabschnitt mit maximaler Axiallänge (maximale Plattendicke) erhält, wobei jeweils zwei unmittelbar benachbarte Segmente derart zueinander ausgerichtet sind, dass sie sich an ihren jeweiligen Zylindermantelabschnitten maximaler Axiallänge flächig axial abstützen oder aufeinander aufliegen, wodurch an der Abstütz- oder Auflagestelle ein Scharnier- oder Gelenkkontakt entsteht. Durch die Keilform der Segmente und die in Axialrichtung fluchtende bzw. angrenzende Anordnung der jeweils benachbarten breitesten Abschnitte der Keilform kann der Relativdrehpunkt der jeweils benachbarten Segmente möglichst weit in den Randbereich mit maximaler Plattendicke verlegt werden, wodurch ein vergleichsweise großer lichter (keilförmiger) Raum zwischen den Spitzen der Keilformen erzeugt wird und ein relativ großer möglicher Kippwinkel zwischen den einzelnen Segmenten erreicht werden kann. Dies resultiert in einem vergleichsweise kleinen Biegeradius des Schwenkmechanismus, sodass im Gegensatz zu prograden Endoskopen, die nur mit einem herkömmlichen Deflecting ausgestattet sind, auch der seitliche Nahbereich durch ein Endoskop mit einer solchen Abklappmechanik bearbeitet und betrachtet werden kann.

Bevorzugt kann das Betätigungselement zur Winkeleinstellung zwischen den Segmenten als zumindest ein Zugelement ausgebildet sein, das mittels Zugkraftbeaufschlagung des am weitesten distal angeordneten keilförmigen Segments die Keilspitzen der Segmente zusammenzieht und so das Abklappen / Abkrümmen des Schwenkmechanismus bewirkt. Bevorzugt kann axial durch die jeweiligen Segmente hindurch zumindest ein Kanal für das zumindest eine Zugelement ausgebildet sein, sodass dieses in einem Innenlumen der Segmente verläuft. Bevorzugte Ausgestaltungen für das Zugelement sind bspw. Seilzüge. Besonders bevorzugt kann das Zugelement als ein Bowdenzug ausgebildet sein, der an dem am weitesten distal und an dem am weitesten proximal angeordnete Segment zumindest mittelbar verankert oder abgestützt ist und diese zum Abklappen / Abkrümmen des Schwenkmechanismus gegeneinander verspannt.

Das Rückstellen in die gerade Position kann bspw. über eine Schubsteifigkeit (wie bei einem schubsteifen Bowdenzug) oder über die Eigenelastizität der Verbindung der Keilsegmente oder auch mit eingearbeitete Federelemente erfolgen. Diese Rückstellung kann im Bedarfsfall auch über die Geradeausstellung etwas hinausgehen um den Sichtbereich zu erweitern und die Endoskopspitze bei Geradeausstellung vorzuspannen und somit zu versteifen

Gemäß einem bevorzugten Aspekt der Erfindung kann zur Fixierung der Stellung des Schwenkmechanismus eine Bandbremse vorgesehen sein, die ein vorzugsweise schubsteifes Bremsband aufweist, das an dem am weitesten distal angeordneten keilförmigen Segment oder am Endoskopkopf fixiert ist und durch die Bereiche mit minimaler axialer Länge (Keilspitzen) der Segmente in Längsrichtung des Endoskops verläuft. Die Bandbremse kann bevorzugt eine proximal der keilförmigen Segmente angeordnete Bremsbacke aufweisen, die in Eingriff mit dem Bremsband bringbar ist, um dieses festzuhalten und somit die Stellung des Schwenkmechanismus zu fixieren. Vorteilhafter Weise kann ein Piezoelement zum Betätigen der Bremsbacke vorgesehen sein.

Gemäß einem zweiten, ggf. unabhängig oder in Kombination mit dem vorstehenden ersten Aspekt beanspruchbaren Aspekt der Erfindung weist der Schwenkmechanismus zumindest zwei, bevorzugt zumindest drei flexible, schubsteife Drähte oder Stangen (Wellen) auf, die innerhalb des langgestreckten Grundkörpers (in einem Randbereich) des Endoskops entlang dessen Längsrichtung verlaufen und relativ zu dem Grundkörper verschiebbar sind. Die Drähte oder Stangen sind dabei kippbar/drehbar an einer proximalen Seite des Endoskopkopfs angelenkt.

In anderen Worten ausgedrückt hat das Endoskop gemäß dem zweiten Aspekt der vorliegenden Erfindung einen Endoskopschaft, ggf. ein distal am Endoskopschaft angeordnetes aktiv betätigbares Deflecting bekannten Aufbaus sowie einen Endoskopkopf am distalen Schaft- oder Deflectingende. Des Weiteren sind wenigstens zwei, vorzugsweise drei biegeflexible, schubsteife Drähte/Stäbe im Endoskopschaft und (falls vorhanden) Deflecting gelagert, welche distal am Endskopkopf bzw. einem Endoskopkopfteil angelenkt/fixiert sind. Die Drähte/Stangen sind dafür vorgesehen und ausgebildet bzw. derart gelagert und montiert, dass sie ausschließlich den Endoskopkopf bzw. das entsprechende (distale) Endoskopkopfteil relativ zudem unmittelbar dazu axial (proximal) benachbarten Endoskopsegement (entspricht quasi einem weiteren proximalen Endoskopkopfteil) bewegen können, in dem sie bevorzugt wahlweise (unabhängig voneinander) vor- und zurückgeschoben werden (und damit aus dem proximalen Endoskopkopfteil herausgefahren und zurückgezogen werden), wobei ausschließlich der Endoskopkopf bzw. das distale Endoskopkopfteil relativ zum (dadurch/dabei unbetätigten) Schaft bewegt/geschwenkt wird.

D.h. die Drähte/Stangen können von der proximalen Seite her über den Endokopschaft hinaus vorgeschoben bzw. eingestellt werden. Durch unterschiedlich weites Vorschieben der einzelnen Drähte oder Stangen axial über den Schaft hinaus werden verschiedene Schwenkstellungen ausschließlich des Endoskopkopfes bzw. distales Kopfteils bezüglich des Schafts bzw. proximales Kopfteil erzielt, ähnlich dem Prinzip eines Stativs mit drei (oder mehr) längenverstellbaren Beinen. Zur Abdichtung zwischen dem (rohrförmigen) Endabschnitt des Schafts bzw. proximalen Kopfteil und dem Endoskopkopf bzw. distalen Kopfteil in der vorgeschobenen/abgeklappten Stellung kann bspw. eine dehnbare Hülle, bevorzugt aus einem Polyurethan oder einem Silikon, ein Faltenbalg oder dergleichen verwendet werden.

Bevorzugt kann eine solche Ausführungsform mit zumindest drei flexiblen Drähten mit einer robotischen Steuerung ausgeführt sein, d.h. die Drähte können bspw. mit Linearmotoren, Hydraulikkolben oder dergleichen und mittels einer entsprechenden Steuersoftware betrieben werden. Ebensogut ist auch eine Bedienung des Schwenkmechanismus bzw. eine Steuerung des Vorschubs der einzelnen Drähte mittels manueller Stellelemente wie Drehknaufen oder dergleichen umsetzbar.

Gemäß einem weiter ggf. unabhängig oder in Kombination mit dem ersten und/oder zweiten Aspekt beanspruchbaren dritten Aspekt der Erfindung kann zum Ermöglichen eines präzisen Ausrichtens des Arbeitskanalausgangs bzw. eines durch diesen geführten minimalinvasiven Instruments zwischen dem Schwenkmechanismus oder dem Endoskopschaft (oder Deflecting) und dem distal zu diesem angeordneten Endoskopkopf/Endokopkopfteil eine zusätzlichen Feinjustierungsvorrichtung bzw. Feinwinkeleinstellung vorgesehen sein.

Gemäß einem bevorzugten Ausführungsbeispiel der Erfindung kann zur Feinwinkeleinstellung ein hydraulisches Stellglied vorgesehen sein, das zwischen dem Schwenkmechanismus und dem Endoskopkopf angeordnet ist und das bevorzugt in einem Randbereich des Endoskopkopfes nahe dem Außenradius der durch den Schwenkmechanismus durchgeführten Schwenkbewegung angeordnet sein kann.

Gemäß einem weiter bevorzugten Ausführungsbeispiel der Erfindung kann die Feinjustierung eine magnetisierbare Biegefeder aufweisen, die in einer maximal gekippten Stellung (ca. 5°- 10° gegenüber der prograden Ausrichtung) vorgespannt ist. Weiter kann die Feinjustierung eine proximal der Biegefeder angeordnete Magnetspule aufweisen, die mit einem Strom beaufschlagt werden kann, um die Biegefeder entgegen ihrer Vorspannrichtung in die prograde Ausrichtung zu ziehen.

Gemäß einem weiter bevorzugten Ausführungsbeispiel der Erfindung kann die Feinjustierung zumindest zwei relativ zueinander um die Endoskopmittelachse verdrehbare Keilringe (keilförmige Ringsegmente) aufweisen, wobei die distale Stirnfläche des distalen Keilrings durch Relativdrehung der beiden Keilringe gegeneinander gegenüber der proximalen Seitenfläche des proximalen Keilrings angestellt werden kann. Dies kann bspw. mittels einer biegeflexiblen, torsionssteifen Antriebswelle bewerkstelligt werden. Der axiale Zusammenhalt der Keilringe kann bspw. über eine Zugfeder oder formschlüssig über Kugelkopfknochenanordnung bewerkstelligt werden.

Gemäß einem weiter bevorzugten Ausführungsbeispiel der Erfindung kann zur Feinjustierung der Endoskopkopf in einer um 5° bis 10° zur Endoskoplängsachse geneigten Position federvorgespannt sein und es kann ein im Randbereich des Biegeaußenradius der Feinjustierung angeordneter Bowdenzug vorgesehen sein, mittels welchem der Kopf in eine prograde Ausrichtung gezogen werden kann, um eine Feinjustierung in besagtem Winkelbereich von 5° bis 10° zu bewerkstelligen.

Gemäß einem weiter bevorzugten Ausführungsbeispiel der Erfindung kann zur Feinjustierung Piezobiegewandler als Teil einer Gelenkverbindung zwischen dem Schwenkmechanismus und dem Endoskopkopf vorgesehen sein. Über eine Variation der Spannung (die über eine entsprechende Leitung von einer Basisstation bereitgestellt wird) ist die Biegung des Piezobiegewandlers und damit der Kippwinkel des Kopfträgers feinjustierbar. Von Vorteil ist dabei, dass der Piezobiegewandler beim Halten seiner Position keine Leistungsaufnahme benötigt und sich folglich nicht erhitzt.

### Kurzbeschreibung der Figuren

Fig. 1A ist eine Darstellung eines allgemeinen Endoskopaufbaus, wie er im Stand der Technik geläufig ist;
Fig. 1B ist eine Darstellung zur Veranschaulichung eines Anwendungsgebietes eines Endoskops mit einem erfindungsgemäßen Schwenkmechanismus nach einem ersten Aspekt der Erfindung;
Fig. 2A ist eine Darstellung einer ersten Ausführungsform des erfindungsgemäßen Schwenkmechanismus nach Fig. 1B in einer Frontansicht;
Fig. 2B ist eine Darstellung der ersten Ausführungsform des erfindungsgemäßen Schwenkmechanismus nach Fig. 1B in einer Seitenansicht;
Fig.3A ist eine perspektivische Darstellung eines erfindungsgemäßen Schwenkmechanismus in einer prograden Ausrichtung nach einem zweiten Aspekt der Erfindung;
Fig. 3B ist eine perspektivische Darstellung des erfindungsgemäßen Schwenkmechanismus nach Fig. 3A in einer um ca. 45° geschwenkten Ausrichtung;
Fig. 4 ist eine perspektivische Darstellung einer Feinjustierung bzw. einer Feinwinkeleinstellung gemäß einem dritten Aspekt der Erfindung;
Fig. 5A ist eine perspektivische Darstellung einer Feinjustierung bzw. einer Feinwinkeleinstellung gemäß einer bevorzugten Abwandlung;
Fig. 5B ist eine Seitenansicht der Feinjustierung bzw. der Feinwinkeleinstellung gemäß Fig. 5A;
Fig. 6A ist eine Seitenansicht Darstellung einer Feinjustierung bzw. einer Feinwinkeleinstellung gemäß einer weiteren Abwandlung in einer prograden Ausrichtung;
Fig. 6B ist eine Seitenansicht der Feinjustierung bzw. der Feinwinkeleinstellung gemäß Fig. 6A in einer abgewinkelten Ausrichtung;
Fig. 7A ist eine Seitenansicht Darstellung einer Feinjustierung bzw. einer Feinwinkeleinstellung gemäß einer weiteren Abwandlung in einer prograden Ausrichtung;
Fig. 7B ist eine Seitenansicht der Feinjustierung bzw. der Feinwinkeleinstellung gemäß Fig. 7A in einer abgewinkelten Ausrichtung;
Fig. 8A ist eine Seitenansicht Darstellung einer Feinjustierung bzw. einer Feinwinkeleinstellung gemäß einer weiteren Abwandlung in einer prograden Ausrichtung; und
Fig. 8B ist eine Seitenansicht der Feinjustierung bzw. der Feinwinkeleinstellung gemäß Fig. 8A in einer abgewinkelten Ausrichtung.

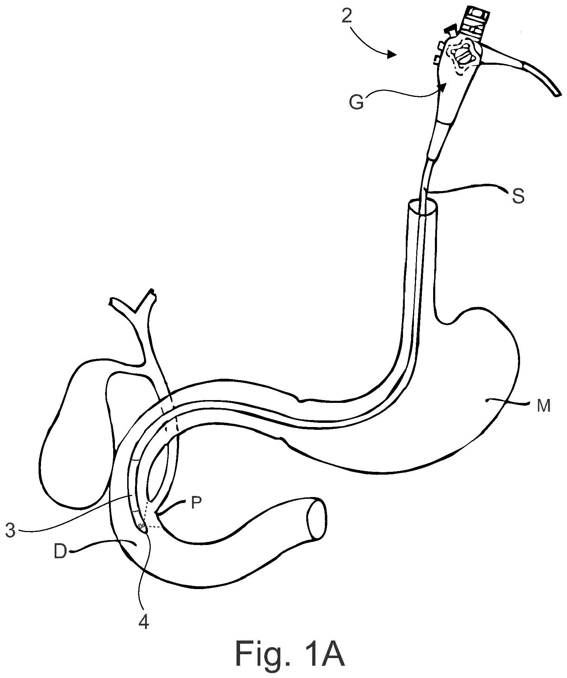

Fig. 1A zeigt einen herkömmlichen Endoskopaufbau aus dem Stand der Technik und dient zur Erläuterung einiger der im Folgenden verwendeten Begriffe. Das Endoskop 2 hat an seinem proximalen, dem Anwender zugewandten Ende ein Griffteil oder Bedienteil G, mittels welchem die Handhabung durch den Anwender erfolgt. Distal an das Griffteil G schließt sich ein langgestreckter Grundkörper des Endoskops 2 an, welcher sich in Richtung von proximal nach distal in eine Reihe konsekutiver Abschnitte mit jeweils unterschiedlicher Funktionalität unterteilen lässt: einen schubsteifen und passiv biegeflexiblen Schaft S, einen aktiv abkrümmbaren distalen Schaftabschnitt 3 (auch Deflectingabschnitt genannt) und eine distale, in der Regel starre, Endkappe auch Endoskopkopf 4 genannt. Das in Figur 1A dargestellte Endoskop 2 ist über die Speiseröhre und den Magen M in den Zwölffingerdarm (Duodenum D) eingeführt, so dass der Endoskopkopf 4 im Bereich vor der Vaterschen Papille liegt.

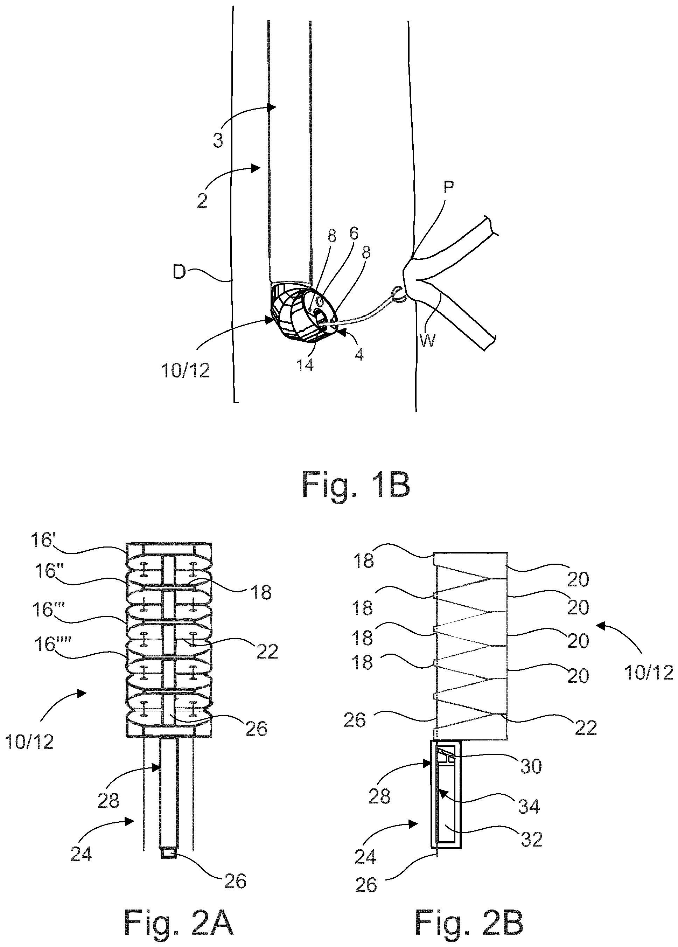

Die Fig. 1B dient der Erläuterung eines bevorzugten Einsatzgebietes eines erfindungsgemäßen Endoskops mit einem Schwenkmechanismus. Zudem zeigt die Fig.1B eine erste bevorzugte Ausführungsform eines solchen Schwenkmechanismus in einem abgewinkelten Zustand.

In Fig. 1B ist schematisch die Vatersche Papille (P) abgebildet, die im rückwärtigen (dorsalen) absteigenden Teil (Pars descendens) des Zwölffingerdarms (Duodenum) (D) lokalisiert und durch die gewundene Geometrie dieses Systems relativ schwer zugänglich ist. Der zur Verfügung stehende Raum im Bereich des Duodenums (D) ist sehr begrenzt, wodurch Eingriffe an der Papilla vateri (P) mit herkömmlichen, prograden Endoskopen nicht möglich sind, da bei einem entsprechenden Abwinkeln der Endoskopspitze in Richtung Eingriffsabschnitt nicht mehr genügend Distanz zum Lumen des Duodenums (D) für eine ordentliche Bildgebung bliebe. Darüber hinaus bestünde bei einem solchen Abwinkeln aufgrund des hohen Biegeradius herkömmlicher Geräte eine Perforationsgefahr im Bereich des Duodenums.

Aus diesem Grund sind dem Stand der Technik die eingangs genannten Duodenoskope bekannt, die eine fest seitwärts bzw. rückblickende Optik sowie einen entsprechend ausgerichteten Arbeitskanal aufweisen, um den zur Verfügung stehenden Raum optimal zu nutzen. Solche Duodenoskope haben jedoch den Nachteil, dass sie in ihrer lateralen/retrograden Ausrichtung der Optik und des Arbeitskanals festgelegt sind. Dies erschwert zum einen die allgemeine Navigation innerhalb des Patienten und macht solche Endoskope zum anderen unflexibel in ihren Einsatzmöglichkeiten. Es handelt sich in anderen Worten um teure Spezialgeräte für ein eng eingeschränktes Anwendungsgebiet.

Ein Grundgedanke der vorliegenden Erfindung ist es deshalb, zusätzlich oder alternativ zu einem bekannten Deflecting einen dem Endoskopkopf zugeordneten Schwenkmechanismus für den Endoskopkopf bzw. ein Endoskopkopfteil bereitzustellen, mittels dessen der Endoskopkopf (Kopfteil) gemeinsam mit der darauf angeordneten Optik und dem Arbeitskanalausgang des Endoskops um mindestens 90° verschwenkbar sind, ohne dass hierzu ein großer Biegeradius benötigt wird. Insbesondere soll eine feinjustierbare Schwenkbewegung ermöglicht werden, um bspw. das Intubieren der Vaterschen Papille zu erleichtern. Gleichzeitig soll ein Multifunktionsgerät bereitgestellt werden, mithilfe dessen der Großteil der gängigen Anwendungen im Bereich des Magen-Darm-Traktes durchgeführt werden können.

Das in Fig. 1B gezeigte Endoskop 2 weist hierfür eine erste beispielhafte Ausführungsform eines erfindungsgemäßen Schwenkmechanismus 10 auf, der in der perspektivischen Darstellung um 90° gegenüber einer prograden/geradeausblickenden Ausrichtung abgekippt ist, um einen Eingriff an der Vaterschen Papille P vorzunehmen. Am distalen Ende des Schwenkmechanismus 10 ist ein Endoskopkopf oder zumindest ein Teil 4 vom Endoskopkopf angeordnet, der/das verschiedene Funktionseinheiten wie eine Optik 6; Leuchtmittel 8 und einen Arbeitskanal 14 aufweist bzw. ausbildet. Der Übersichtlichkeit halber sind in der Fig. 1B und im Folgenden nur die oben genannten nötigsten Funktionseinheiten dargestellt, selbstverständlich kann ein erfindungsgemäßer Endoskopkopf im Bereich des dargestellten Teils 4 zusätzlich diverse andere dem Stand der Technik bekannte Funktionseinheiten, etwa Reinigungsdüsen für ein Objektiv der Optik 6, Absaugkanäle etc. aufweisen.

Der in den Figuren 2A und 2B in einer Front bzw. einer Seitenansicht dargestellte Schwenkmechanismus 10 weist einen Grundkorpus 12 mit einer Anzahl in Axialrichtung des Endoskops 2 aufeinanderfolgend angeordneter Segmente 16 (16', 16", 16‴ und 16"") auf. Dabei sei an dieser Stelle darauf hingewiesen, dass zumindest das distal letzte Segment 16' (oder alle weiteren Segmente) auch als ein proximales Teil des Endoskopkopfs 4 betrachtet werden kann, da sie sich konstruktiv und funktional vom übrigen Schaft und vom proximal ggf. angeordneten Deflecting (falls vorhanden) unterscheiden.

Die einzelnen Segmente 16 haben grundsätzlich eine keilartige Form, d.h. ihre distalen und/oder proximalen Stirnflächen laufen in einem spitzen Winkel aufeinander zu und treffen sich (in Seitenansicht gesehen) in einer Keilspitze 18. Entsprechend ist die axiale Erstreckung der einzelnen Segmente 16 in einem von der Keilspitze 18 abgewandten (diametral gegenüberliegenden) Abschnitt 20 am größten.

Wie insbesondere aus der Fig. 2B ersichtlich ist, sind die beiden Stirnseiten im Bereich maximaler Segmentdicke über einen gegenüber dem Keilbereich (zwischen Spitze und maximaler Segmentdicke) kleinen Teilkreisabschnitt hinweg abgeflacht (im Wesentlichen parallel zueinander ausgerichtet) und bilden so eine Aufstands-/Anlagefläche zur Abstützung zweier benachbarter Segmente bei prograder Schaftausrichtung.

Die einzelnen Segmente 16 sind bevorzugt in der dargestellten Ausführungsform zudem kreiszylinderförmig, um einen kreisrunden Querschnitt längs des gesamten Endoskops 2 zu erzielen. Wie in Fig. 2B gut zu sehen ist, fluchten die Keilspitzen 18 und die von diesen abgewandten Abschnitte 20 in der aufgerichteten / gestreckten Stellung des Schwenkmechanismus 10. Dies hat zur Folge, dass der Schwenkmechanismus 10 unidirektional abwinkelbar ist.

Des Weiteren ist aus der Fig. 1B gut zu erkennen, dass der in den Segmenten 16 ausgebildete Arbeitskanal dezentral zumindest teilweise im Bereich der Segmente mit maximaler Segmentdicke ausgeformt ist, d.h. möglichst nahe jenes Umfangsabschnitts des jeweiligen Segments liegt, das der Segmentspitze diametral gegenüberliegt.

In der in Fig. 2A dargestellten, aufgerichteten/prograden Konfiguration des Schwenkmechanismus 10 grenzen die von den Keilspitzen 18 und abgewandten Abschnitte 20 mit maximaler Segmentdicke jeweils aneinander an und bilden Abstütz- oder Auflagestellen/-flächen 22, um/an welche(n) die einzelnen Segmente 16 relativ zueinander verkippbar sind. Durch die Keilform der Segmente 16 sind in der in der Fig. 2 dargestellten, aufgerichteten Stellung zu den Keilspitzen 18 hin sägezahnartige Freiräume im Grundkorpus 12 des Schwenkmechanismus 10 ausgebildet. Die um die Abstütz- oder Auflagestellen 22 kippbaren Segmente 16 können also unter Verringerung dieser Freiräume aufeinander zu geklappt werden, bis die jeweils proximalen und distalen Stirnflächen benachbarter Segmente 16, wie in der Fig. 1B dargestellt ist, in einer maximal abgekrümmten Stellung aneinander anliegen. Dadurch, dass die Keilspitzen 18 eine relativ geringe Axialerstreckung von hier weniger als 1 mm aufweisen, lässt sich um 90° abgeklappte Stellung mit einem vergleichsweise geringen Biegeinnenradius erzielen. Der geringe Biegeinnenradius hat den Vorteil, dass Optik 6 und Leuchtmittel 8 in besagter abgeklappter Stellung nicht weit über den Außenumfang des Endoskops hinausragen (siehe Fig. 1B).

Um das vorstehend beschriebene Abkrümmen bzw. Abklappen zu bewirken, sind in den Segmenten 16 hier nicht näher dargestellte Bowdenzug-Kanäle vorgesehen, in welchen ebenfalls nicht dargestellte Bowdenzüge geführt sind. Diese sind dazu ausgebildet, das am weitesten distal angeordneten Segment 16' mit einer Zugkraft zu beaufschlagen, wodurch ein Drehmoment in den Abstütz- oder Auflagestellen 22 erzeugt wird, welches ein Abklappen / Abkrümmen des Schwenkmechanismus 10 bewirkt. Entsprechende Bowdenzugmechanismen und zugehörige Bedienelemente sind im Stand der Technik hinlänglich bekannt und werden an dieser Stelle nicht näher erläutert.

Als Schwenkscharnier kann bevorzugt ein in den Arbeitskanal eingesetzter flexibler oder elastischer Schlauch dienen, der die lose aneinander gelegten Segmente schwenkbar miteinander verbindet. Alternativ hierzu ist es aber auch möglich, auf einen solchen Schlauch zu verzichten und stattdessen Drähte im Scharnierbereich der Segmente einzuziehen, welche die Scharniere axial aneinanderhalten, wie dies in der Fig. 2A angedeutet ist.

In der dargestellten bevorzugten Ausführungsform weist der Schwenkmechanismus 10 zusätzlich eine Bandbremse 24 auf, mit welcher sich die Stellung desselben fixieren lässt. Die Bandbremse 24 gemäß der in den Figuren 2A und 2B dargestellten Ausführungsform hat ein schubsteifes, biegeflexibles Bremsband 26 das entlang des Biegeinnenradius des Schwenkmechanismus 10 die Segmente 16 im Bereich ihrer Keilspitzen 18 durchläuft. Das Bremsband 26 ist dabei an dem den Endoskopkopf 4 tragenden und am weitesten distal angeordneten Segment 16' fixiert. Wird der Schwenkmechanismus 10 mittels der Bowdenzüge abgewinkelt/abgekippt, verschiebt sich das Bremsband 26 axial in proximale Richtung und durchläuft dabei eine proximal an den Schwenkmechanismus angrenzende Bremseinrichtung 28. Die Bremseinrichtung 28 weist eine Bremsbacke 30 auf, die als Festkörpergelenk ausgebildet ist. Im dargestellten Beispiel ist die Bremsbacke 30 in der Art eines elastischen Vorsprungs ausgeführt, der integral im Bereich der Bremseinrichtung 28 angeformt ist und schräg zwischen der Radialrichtung und der distalen Axialrichtung des Endoskops 2 verläuft. Das Bremsband 26 verläuft zwischen einem freien Endabschnitt der Bremsbacke 30 und einem Wandabschnitt 34 der Bremseinrichtung 28. Die Bremsbacke 30 ist mit einem in Axialrichtung wirkenden Piezoelement 32 verbunden, das, wenn es mit einer Spannung beaufschlagt wird, die Bremsbacke 30 gen proximal in eine im Wesentlichen in radiale Ausrichtung zieht und in dieser Stellung das Bremsband 28 gegen den Wandabschnitt 34 klemmt und somit den Schwenkmechanismus 10 sperrt. Beim Verlängern des Piezoelements 32 wird das Bremsband 28 freigegeben und der Schwenkmechanismus 10 kann bewegt werden.

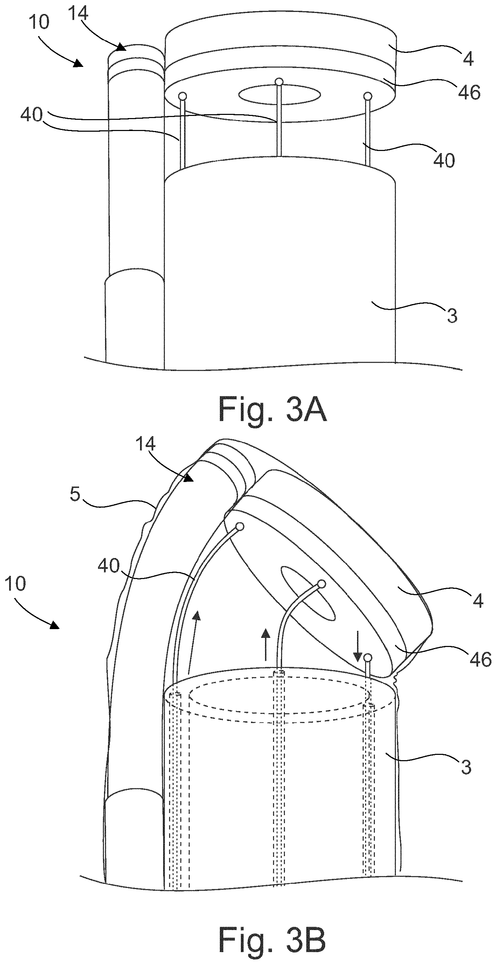

In den Figuren 3A und 3B ist eine zweite bevorzugte Ausführungsform eines Schwenkmechanismus 10 dargestellt. In dieser Ausführungsform ist eine Trägerscheibe 46 vorzugsweise als ein distales Teil des Endoskopkopfs vorgesehen, die an ihrer gen distal gewandten Oberfläche einen Funktionsträger 4 (zur Aufnahme von z.B. einer Optik und Beleuchtung sowie eines Arbeitskanal-Ausgangs) des Endoskopkopfs aufweist. Die Trägerscheibe 46 ist über eine Anzahl (hier dreier) schubsteifer, biegeflexibler Antriebswellen 40 (Drähte/Stangen, welche als proximaler Bestandteil des Endoskopkopfs betrachtet werden können) mit einem aktiv abkrümmbaren Abschnitt 3 (Deflecting) des Endoskopschaftes oder, falls dieser nicht vorhanden ist, mit dem Endoskopschaft selbst verbunden. Die Antriebswellen sind jeweils in einem Randbereich der Trägerscheibe 46, an deren proximal ausgerichteten Seite angelenkt/fixiert. Durch eine Steuerung des Vorschubs der einzelnen Antriebswellen 40 bzw. durch Einstellen deren jeweiligen Überstandes über den distalen Rand (distale Stirnseite) des aktiv abkrümmbaren Abschnitts 3 oder des Endoskopschafts, lassen sich, wie in Fig. 3B zu erkennen ist, verschiedene Kippstellungen der Trägerscheibe 46 bezüglich der distalen Stirnseite des Deflecting/Endoskopschafts einstellen. Die Verwendung dreier Antriebswellen 40 gemäß der dargestellten bevorzugten Ausführungsform hat den Vorteil, dass statische Überbestimmungen vermieden werden. In der dargestellten Ausführungsform sind die Antriebswellen 40 mit einer robotischen Steuerung ausgeführt sind, d.h. sie werden mittels kleiner Motoren (nicht dargestellt) und per Softwaresteuerung bedient. Ebenso gut ist auch eine Bedienung des Schwenkmechanismus bzw. eine Steuerung des Vorschubs der einzelnen Drähte mittels manueller Stellelemente wie Drehknaufe oder dergleichen Handhaben umsetzbar. Zur Abdichtung zwischen dem aktiv abkrümmbaren Abschnitt 3 oder Endoskopschaft und dem Endoskopkopfteil 4 bzw. der Trägerscheibe 46 in der in Fig. 3B dargestellten maximal vorgeschobenen Stellung ist eine dehnbare Hülle 5, bevorzugt aus einem Polyurethan oder einem Silikon, ein Faltenbalg oder dergleichen vorgesehen.

Der Arbeitskanal 14 ist in dem in Figur 3A bzw. 3B dargestellten Beispiel radial außerhalb des Endoskopgrundkörpers (des Schafts S, des Deflectings 3 und des Endoskopkopfes 4) verlaufend angeordnet, also möglichst weit außen bezüglich der beabsichtigen Schwenkrichtung. Dies hat den Vorteil, dass beim Abwinkeln des Schwenkmechanismus 10, wie in Figur 3B gezeigt ist, kleinere Abklapp- bzw. Schwenkradien bei ausreichend großen Krümmungsradien des Arbeitskanals erzielt werden können (wie auch beim Ausführungsbeispiel gemäß dem vorstehend beschriebenen ersten Aspekt der Erfindung), was wiederum das Durchschieben minimalinvasiver Werkzeuge vereinfacht.

Herkömmliche Endoskope für Eingriffe an der Vaterschen Papille (sogenannte Duodenoskope) weisen einen im distalen Bereich eines prograd ausgerichteten Arbeitskanalausgang angeordneten aktiv betätigbaren Umlenkhebel (auch Albarranhebel genannt) auf, mittels welchem sich durch den Arbeitskanal vorgeschobene minimalinvasive Instrumente, Führungsdrähte und dergleichen gezielt Umlenken lassen, wohingegen die Ausrichtung der Optik fix vorgegeben ist und sich in der Regel nicht verändern lässt.

Die erfindungsgemäßen Schwenkmechanismen 10 nach den vorstehend beschriebenen Ausführungsbeispielen, machen den vorgenannten Albarranhebel obsolet, da minimalinvasive Instrumente, Führungsdrähte und dergleichen durch die Ausrichtbarkeit des Arbeitskanalausgangs zusammen mit der Optik auf engstem Raum per se umlenkbar sind. Es ist, wie eingangs ausgeführt wurde, von Vorteil, wenn der Arbeitskanal 14 an der Außenseite bzw. radial außenliegend zwischen Schaft S und Umhüllung 5 im Bereich maximaler Segmentdicke angeordnet ist, sodass dieser in bei einem Abkrümmen des Schwenkmechanismus 10 im Außenradius der Biegung gelegen sein kann und so den maxmal möglichen Abklappweg möglichst wenig beeinflusst. Je nach gewählter Ausführungsform des Schwenkmechanismus 10 kann jedoch zusätzlich eine Feinjustierung der Verschwenkung/Abwinkelung von Vorteil sein, um bspw. die Vatersche Papille zielgerichtet zu intubieren.

Gemäß einem weiteren und ggf. unabhängig zu beanspruchenden Aspekt der Erfindung kann deshalb zusätzlich zur genannten Schwenkeinrichtung 10 eine Feinjustierungsvorrichtung (Feinjustierung bzw. Feinwinkeleinstellung) 52 vorgesehen sein, die gemäß der bevorzugten Ausführungsform zwischen Schwenkeinrichtung 10 und Endoskopkopf 4 vorgesehen ist. Die nachfolgenden Feinjustierungen werden jeweils beispielhaft in der Anordnung auf einem Segment 16' gemäß der in den Figuren 1, 2A und 2B dargestellten ersten Ausführungsform der Schwenkeinrichtung dargestellt. Selbstverständlich ist eine Kombination mit den anderen Ausführungsformen für eine Schwenkeinrichtung ebenso möglich und vorgesehen.

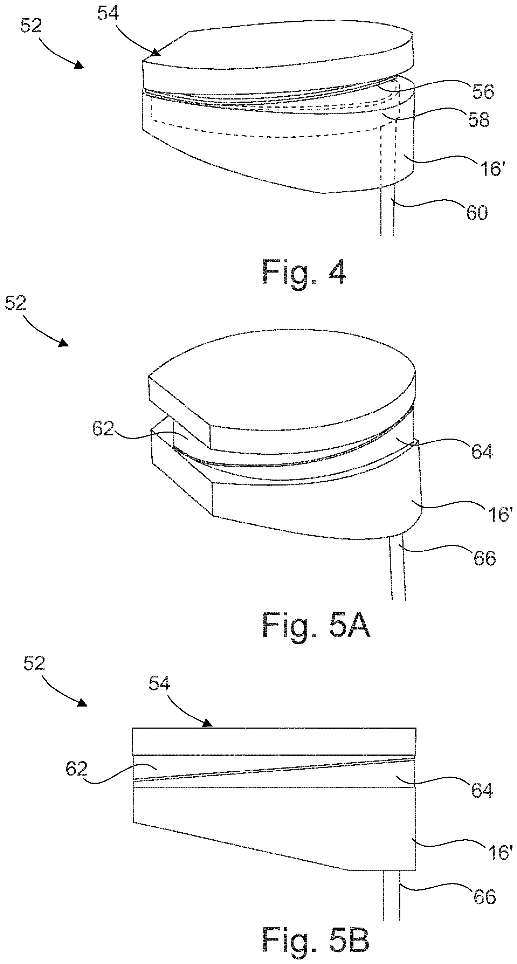

Fig. 4 zeigt eine Ausführungsform der Erfindung mit einer Feinjustierung 52. Die dargestellte Feinjustierung weist einen scheiben- bzw. plattenförmigen Kopfträger 54 auf, der mittels einer magnetisierbaren Biegefeder 56 in einer maximal gekippten Stellung (ca. 5°- 10° gegenüber der prograden Ausrichtung) vorgespannt ist. Eine proximal der Biegefeder 56 in einem Segment 16' der Schwenkeinrichtung 10 angeordnete Magnetspule 58 ist über eine Stromzuführung 60 mit einer nicht dargestellten Basisstation verbunden. Über eine Variation des durch die Magnetspule 58 fließenden Stroms, kann der Kopfträger 54 entgegen der Vorspannung zur Magnetspule 58 hingezogen werden. Bevorzugt kann zum Halten der feinjustierten Ausrichtung ein separates Fixierelement (nicht dargestellt) verwendet werden, um eine übermäßige Spulenerwärmung zu vermeiden.

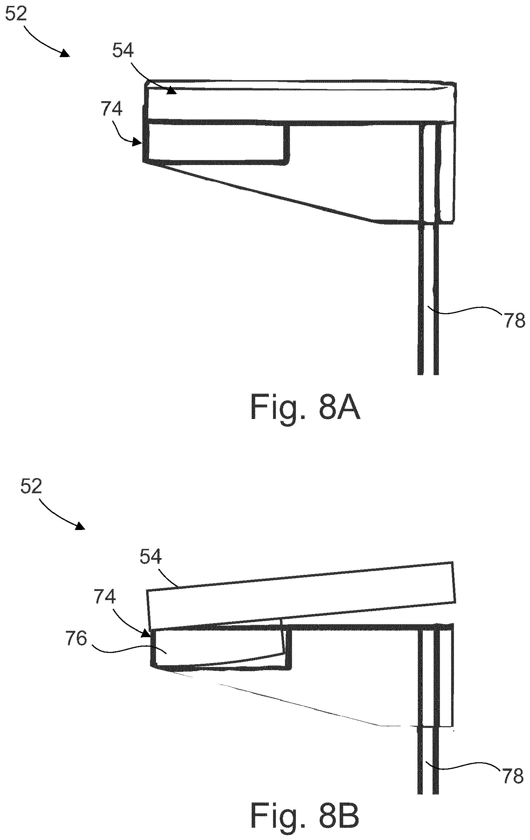

Figuren 5A und 5B zeigen eine weitere Ausführungsform der Erfindung mit einer Feinjustierung 52. Bei der dargestellten weitere Ausführungsform der Feinjustierung 52 ist der Kopfträger 54 über zwei relativ zueinander um die Endoskopmittelachse verdrehbare Keilringe 62, 64 mit einem Segment 16' der Schwenkeinrichtung 10 verbunden. In der Figuren 8A und 8B sind die Keilringe 62, 64 in einer schnittsymetrischen Ausrichtung dargestellt, wodurch eine Geradeausstellung der Kopfträgers 4 bewirkt wird. Verdreht man nun bspw. den distalen Keilring 62 gegenüber dem proximalen Keilring 64, so kippt die distale Stirnfläche des distalen Keilrings(und somit der Kopfträger 54) gegenüber der Endoskoplängsachse. Die Relativdrehung wird im dargestellten Beispiel mittels einer biegeflexiblen, torsionssteifen Antriebswelle 66 bewirkt, an deren distalen Endbereich ein nicht dargestelltes Ritzel angeordnet ist, das mit einem Zahnkranz am distalen Keilring 62 zusammenwirkt. Der axiale Zusammenhalt der Keilringe 62, 64 kann bspw. über eine Zugfeder, Federbügel, Kugelkopfknochenanordnung oder vergleichbare formschlüssige Anordnungen bewerkstelligt werden. Alternativ kann die Antriebswelle 66 zugfest an dem distalen Keilring 62 fixiert sein, um diesen an den proximalen Keilring 64 heranzuziehen. Diese Ausführungsform hat den Vorteil, dass eine sehr gute Steifigkeit der feinjustierten Positionierung erzielbar ist. Die vorstehend beschriebene Anordnung kann auch gedoppelt werden, d.h. es können mindestens vier Keilringe vorgesehen werden, die abwechselnd zueinander fixiert bzw. drehbar sind und die mittels zweier Antriebswellen 66 gegeneinander verdreht werden können. Eine solche Anordnung ermöglicht ein Schwenken in alle Richtungen. Der in Fig. 8B einsehbare freie Innenraum der Keilringe 62, 64 bietet ausreichend Platz für Kanäle und Leitungen.

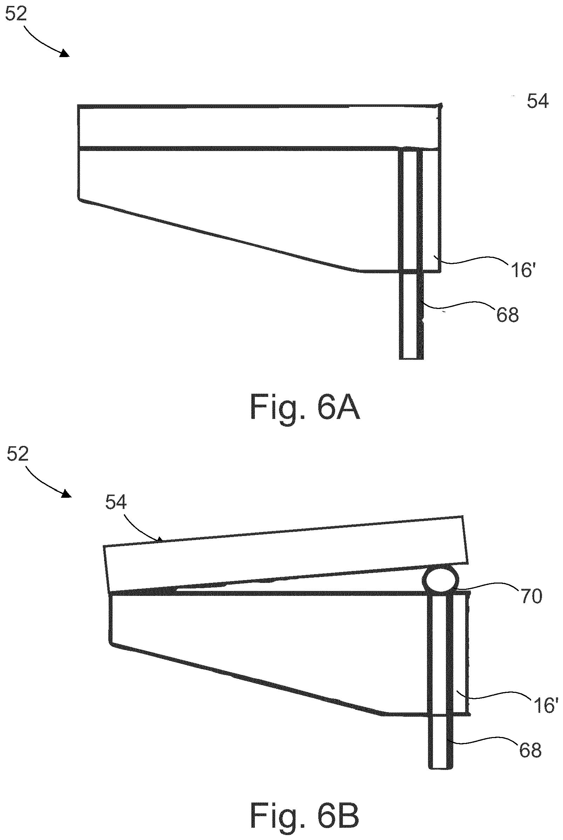

Figuren 6A und 6B zeigen eine weitere Ausführungsform der Erfindung mit einer Feinjustierung 52. In dieser wird der Kopfträger 54 durch Federvorspannung in der prograden Stellung gehalten. Umfangsseitig im Bereich des Biegeinnenradius des Schwenkmechanismus 10 ist der Kopfträger 54 schwenkbar am Segment 16' angeordnet. An einem gegenüberliegenden Randabschnitt ist ein in Axialrichtung des Endoskops verlaufender Hydraulikschlauch 68 vorgesehen der mit seinem distalen Ende unterhalb des Kopfträgers 54 endet. An besagtem distalen Ende des Hydraulikschlauches 68 ist ein Ballon- oder Kolbenelement 70 angeordnet, welches bei Druckbeaufschlagung ein variables Kippen des Kopfträgers 54 bewerkstelligt. Ein solches System bietet den Vorteil einer guten Steifigkeit der feineingestellten Position

Zudem hat ein mit Wasser befüllter Hydraulikschlauch 68 den Vorteil einer guten Biokompatibilität, was Risiken für den Patienten bei einer Fehlfunktion minimiert. In Kombination mit der ersten Ausführungsform des Schwenkmechanismus 10 (Figuren 1, 2A und 2B) mit keilförmigen Segmenten 16, kann der Hydraulikschlauch 68 zugleich als Kippgelenk für die Segmente 16 verwendet werden und erfordert somit keinen zusätzlichen Platz.

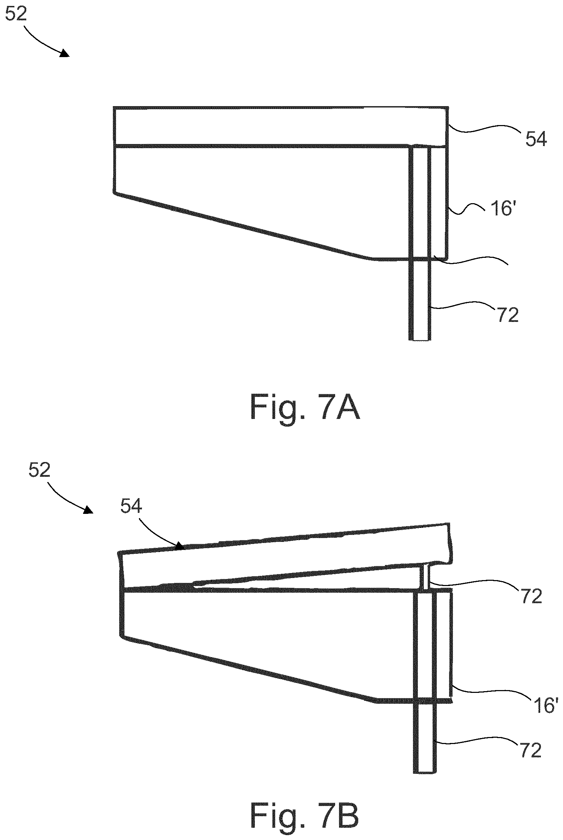

Figuren 7A und 7B zeigen eine weitere Ausführungsform der Erfindung mit einer Feinjustierung 52. Diese funktioniert ähnlich zur unmittelbar vorgenannten Ausführungsform der Feinjustierung allerdings ist der Kopfträger 54 hier in seiner Endposition vorgespannt (bspw. 5° bis 10° zur Endoskoplängsachse geneigt). Ein im Randbereich des Biegeaußenradius des Schwenkmechanismus 10 angeordneter Bowdenzug 72 dient dazu den Kopfträger 54 entgegen der Vorspannung in Richtung hin zum keilförmigen Segmenten 16' zu ziehen. Wegen des Bowdenzugs 72 hat auch eine solche Feinjustierung 52 eine gute Steifigkeit und keine Zulassungsprobleme für den medizinischen Bereich, da der Einsatz von Bowdenzügen in der Endoskopie bewährt ist. In Kombination mit der ersten Ausführungsform des Schwenkmechanismus 10 (Figuren 1, 2A und 2B) mit keilförmigen Segmenten 16, kann der Bowdenzug 72 zugleich als Kippgelenk für die Segmente 16 verwendet werden und erfordert somit keinen zusätzlichen Platz.

Figuren 8A und 8B zeigen eine weitere Ausführungsform der Erfindung mit einer Feinjustierung 52. In dieser Ausführungsform dient ein im Bereich des Biegeinnenradius des Schwenkmechanismus angeordneter Piezobiegewandler 74 als Gelenkverbindung zwischen dem Schwenkmechanismus 10 (dem Segment 16') und dem Kopfträger 54. In der prograden Normalstellung wird der Kopfträger 54 durch das Piezoelement 76 des Piezobiegewandlers 74 in Position gehalten. Über eine Variation der Spannung (übertragen über Leitung 78) ist die Biegung des Piezobiegewandlers 74 und damit der Kippwinkel des Kopfträgers 54 feinjustierbar. Von Vorteil ist dabei, dass der Piezobiegewandler 74 beim Halten seiner Position keine Leistungsaufnahme benötigt und sich folglich nicht erhitzt. Diese Ausführungsvariante kommt zudem vorteilhafter Weise auch ohne Federvorspannung aus.

### Bezugszeichen

- 2: Endoskop;
- 3: distaler Endabschnitt des Endoskopschaftes / aktiv abkrümmbarer Abschnitt
- 4: Endoskopkopf;
- 5: Hülle
- 6: Optik;
- 8: Leuchmittel;
- 10: Schwenkmechanismus;
- 12: Grundkorpus;
- 14: Arbeitskanal;
- 16: Segment;
- 18: Zylindermantelabschnitt mit minimaler Axiallänge / Keilspitze;
- 20: Zylindermantelabschnitt mit maximaler Axiallänge / von der Keilspitze abgewandter Abschnitt;
- 22: Abstütz- oder Auflagestelle bzw. Gelenkabschnitte;
- 24: Bandbremse;
- 26: Bremsband;
- 28: Bremseinrichtung;
- 30: Bremsbacke;
- 32: Piezoelement;
- 34: Wandabschnitt;
- 46: Trägerscheibe;
- 52: Feinjustierung;
- 54: Kopfträger;
- 56: Biegefeder;
- 58: Magnetspule;
- 60: Stromzuführung;
- 62: distaler Keilring;
- 64: proximaler Keilring;
- 66: Antriebswelle;
- 68: Hydraulikschlauch;
- 70: Ballon- oder Kolbenelement;
- 72: Bowdenzug;
- 74: Piezobiegewandler;
- 76: Piezoelement;
- 78: Leitung;
- D: Duodenum;
- M: Magen;
- P: Papilla Vateri;
- S: Schaft; und
- W: Werkzeug.

## Patentansprüche

1. Endoskop (2) mit einem Endoskopkopf (4) und wahlweise einem distalen Teil des Endoskopkopfs (4), wobei der Endoskopkopf (4) an einem, vorzugsweise aktiv abkrümmbaren, distalen Endabschnitt eines Endoskopschaftes (3), angeordnet ist und der Endoskopkopf (4) oder der distale Teil des Endoskopkopfs (4) zumindest eine Optik (6) zur Bildgebung aufweist oder trägt, und
einem Arbeitskanal (14), der sich integral in dem Endoskop (2) längserstreckt und dessen Arbeitskanalausgang im Endoskopkopf (4) oder im distalen Teil des Endoskopkopfs (4) angeordnet oder ausgebildet ist,
und einem Schwenkmechanismus (10), der zwischen dem distalen Schaft-Endabschnitt und dem Endoskopkopf (4) oder distalen Teil des Endoskopkopfs (4) angeordnet ist und der dazu ausgebildet ist, den Endoskopkopf (4) oder distalen Teil des Endoskopkopfs (4) abklapp- oder abwinkelbar zu halten, wofür der Schwenkmechanismus (10) zumindest ein Segment (16) hat, das eine in Draufsicht im Wesentlichen runde und in Seitenansicht im Wesentlichen keilförmige Scheibe oder Platte ausbildet, die in Seitenansicht betrachtet einen flachen Umfangsabschnitt (18) aufweist und die sich in Richtung hin zum diametral gegenüberliegenden Umfangsabschnitt keilförmig verdickt, um an diesem gegenüberliegenden Umfangsabschnitt ein Plattendicken-Maximum zu erhalten, welches sich über einen Teilumfangsabschnitt des Plattensegments (16) erstreckt, wodurch sich eine Segment-Aufstands- oder -Abstützfläche (22) ergibt, die im Wesentlichen senkrecht zur Platten-Mittelachse ausgerichtet ist und wobei in Draufsicht gesehen der Arbeitskanal dezentral zumindest teilweise in diesem Teilumfangsabschnitt mit maximaler Plattendicke ausgebildet ist.

2. Endoskop (2) gemäß Anspruch 1, **dadurch gekennzeichnet, dass** der Schwenkmechanismus (10) eine Mehrzahl axial aufeinanderfolgender und mittels zumindest eines Betätigungselements aktiv gegeneinander winkelverstellbarer Segmente (16) aufweist, die als die keilförmigen Platten mit keilförmig zueinander ausgerichteten Stirnseiten ausgebildet sind, wodurch jede Platte einen Zylindermantelabschnitt mit minimaler Axiallänge (18) und einen gegenüberliegenden Zylindermantelabschnitt mit maximaler Axiallänge (20) erhält, wobei jeweils zwei unmittelbar benachbarte Segmente (16) derart zueinander ausgerichtet sind, dass sie sich an ihren jeweiligen Zylindermantelabschnitten maximaler Axiallänge (20) axial abstützen oder aufeinander aufliegen, wodurch an der Abstütz- oder Auflagefläche oder-stelle (22) ein Scharnier- oder Gelenkkontakt entsteht.

3. Endoskop (2) gemäß Anspruch 1, **dadurch gekennzeichnet, dass** der Zylindermantelabschnitt mit minimaler Axiallänge (18) der jeweiligen Segmente (16) eine Axiallänge kleiner als 2 mm, bevorzugt kleiner als 1 mm hat, wodurch dieser in einer Seitenansicht betrachtet eine Keilspitze (18) ausbildet.

4. Endoskop (2) gemäß dem Oberbegriff des Anspruchs 1, **dadurch gekennzeichnet, dass** der Schwenkmechanismus (10) wenigstens zwei, vorzugsweise drei biegeflexible, schubsteife Drähte Bowdenzüge oder Stäbe (40) hat, welche im Endoskopschaft (3) gelagert sind und welche distal am Endskopkopf (4) oder einem distalen Teil des Endoskopkopfs (4) angelenkt oder fixiert sind, der ausschließlich über die Drähte Bowdenzüge oder Stäbe (40) am Endoskopschaft (3) gehalten ist, wobei die Drähte oder Stäbe (40) dafür vorgesehen und ausgebildet sind, dass sie ausschließlich den Endoskopkopf oder das distale Endoskopkopfteil (4) relativ zu dem unmittelbar dazu axial proximal benachbarten Endoskopsegement oder Endoskopkopfteil bewegen können, in dem sie bevorzugt wahlweise sowie unabhängig voneinander vor- und zurückgeschoben werden, wodurch ausschließlich der Endoskopkopf oder das distale Teil des Endoskopkopfs (4) relativ zum Schaft bewegt, insbesondere geschwenkt wird.

5. Endoskop (2) gemäß einem der Ansprüche 1 bis 4, **dadurch gekennzeichnet, dass** das Endoskop (2) zusätzlich zum Schwenkmechanismus (10) eine zu diesem separat ausgebildete Feinjustierungsvorrichtung (52) zum Ermöglichen eines präziseren Ausrichtens des Arbeitskanalausgangs aufweist.

6. Endoskop (2) gemäß Anspruch 5, **dadurch gekennzeichnet, dass** die Feinjustierungsvorrichtung (52) ein hydraulisch betätigtes Stellglied (70) ist, das über einen Schlauch (68) mit einem Druck beaufschlagbar ist, um ein Neigen des Endoskopkopfes (4) zu bewirken.

7. Endoskop (2) gemäß Anspruch 6, **dadurch gekennzeichnet, dass** die Feinjustierungsvorrichtung (52) mittels eines, insbesondere magnetisierbaren Federelementes (56) in einer maximal gekippten Stellung von weniger als 15° gegenüber der Endoskoplängsachse vorgespannt ist und mittels einer strombeaufschlagbaren Magnetspule (58) oder eines am Endoskopkopf (4) fixierten und zugkraftbeaufschlagbaren Zugdrahtes (72) entgegen seiner Vorspannungsrichtung in eine prograde Ausrichtung überführbar ist.

8. Endoskop (2) gemäß Anspruch 7, **dadurch gekennzeichnet, dass** die Feinjustierungsvorrichtung (52) zumindest zwei aneinander anliegende und abgleitende sowie relativ zueinander konzentrisch verdrehbare Keilringe (62, 64) aufweist.

9. Endoskop (2) gemäß Anspruch 6, **dadurch gekennzeichnet, dass** die Feinjustierung (52) einen Piezobiegewandler (74) aufweist, der über eine elektrische Leitung (78) mit einer Spannung beaufschlagbar ist.

10. Endoskop (2) nach einem der vorstehenden Ansprüche, **dadurch gekennzeichnet, dass** es ein Kombiniertes Gastro-Duodenoskop der Einweggerät-Bauart ist.

## Claims

1. An endoscope (2) having an endoscope head (4) and optionally a distal part of the endoscope head (4), the endoscope head (4) being arranged on a preferably actively curvable, distal end portion of an endoscope shaft (3), and the endoscope head (4) or the distal part of the endoscope head (4) having or supporting at least one optics device (6) for imaging, and
a working channel (14) which extends integrally within the endoscope (2) in the longitudinal direction and having a working channel exit which is arranged or formed in the endoscope head (4) or in the distal part of the endoscope head (4),
and a pivot mechanism (10) arranged between the distal shaft end portion and the endoscope head (4) or the distal part of the endoscope head (4) and configured to hold the endoscope head (4) or the distal part of the endoscope head (4) in a foldable or bendable manner, for which the pivot mechanism (10) has at least one segment (16) which defines a disk or plate which is substantially round in plan view and substantially wedge-shaped in side view, said disk or plate having a flat circumferential portion (18) when seen in side view, and which thickens in wedge-shaped manner towards the diametrically opposite circumferential portion to obtain a plate thickness maximum at said opposite circumferential portion, which extends over a partial circumferential portion of the plate segment (16), resulting in a segment support or abutment surface (22) which is oriented substantially perpendicular to the plate central axis and wherein, as seen in plan view, the working channel is formed to be decentral at least partially in said partial circumferential section of maximum plate thickness.

2. The endoscope (2) according to claim 1, **characterized in that** the pivot mechanism (10) has a plurality of axially successive segments (16) which are actively adjustable relative to one another in their angle by means of at least one actuating element, which are formed as the wedge-shaped plates having end faces oriented in wedge-shaped manner relative to one another, as a result of which each plate is given a cylindrical envelope portion of minimum axial length (18) and an opposite cylindrical envelope portion of maximum axial length (20), wherein two directly adjoining segments (16) are aligned in each case relative to one another such that they are axially supported or rest upon each other at their respective cylindrical envelope portions of maximum axial length (20), resulting in a hinge or joint contact on the support or abutment surface or area (22).

3. The endoscope (2) according to claim 1, **characterized in that** the cylindrical envelope portion of minimum axial length (18) of the respective segments (16) has an axial length of less than 2 mm, preferably less than 1 mm, which is why it defines a wedge tip (18) as seen in side view.

4. The endoscope (2) according to the preamble of claim 1, **characterized in that** the pivot mechanism (10) has at least two, preferably three resiliently flexible, shear-resistant wires, Bowden cables or rods (40) which are supported in the endoscope shaft (3) and are distally articulated or fixed on the endoscope head (4) or a distal part of the endoscope head (4), which is retained on the endoscope shaft (3) only via the wires, Bowden cables or rods (40), the wires or rods (40) being provided and configured to be able to move only the endoscope head or the distal endoscope head part (4) relative to the directly and axially proximally adjoining endoscope segment or endoscope head part, wherein the wires, Bowden cables or rods (40) are pushed back and forth preferably selectively as well as independently of one another, which is why only the endoscope head or the distal part of the endoscope head (4) is moved, in particular pivoted relative to the shaft.

5. The endoscope (2) according to any of claims 1 to 4, **characterized in that** the endoscope (2), in addition to the pivot mechanism (10), comprises a fine adjustment device (52) which is formed to be separate from the pivot mechanism and allows a more precise alignment of the working channel exit.

6. The endoscope (2) according to claim 5, **characterized in that** the fine adjustment device (52) is a hydraulically actuated actuator (70) to which a pressure can be applied via a hose (68) in order to bring about an inclination of the endoscope head (4).

7. The endoscope (2) according to claim 6, **characterized in that** the fine adjustment device (52) is biased by means of a, particularly magnetizable, spring element (56) into a maximum tilted position of less than 15° with respect to the longitudinal axis of the endoscope and can be transferred against its biasing direction into a prograde alignment by means of a solenoid (58), which can be supplied with electrical current, or by means of a pull wire (72) which is fixed on the endoscope head (4) and to which a tensile force can be applied.

8. The endoscope (2) according to claim 7, **characterized in that** the fine adjustment device (52) has at least two wedge rings (62, 64), which rest against and slide on each other and are concentrically rotatable relative to one another.

9. The endoscope (2) according to claim 6, **characterized in that** the fine adjustment (52) has a piezo-based flexural transducer (74) to which a voltage can be applied via an electrical line (78).

10. The endoscope (2) according to any of the preceding claims, **characterized in that** the endoscope (2) is a combined gastroscope-duodenoscope of the single-use device type.

## Revendications

1. Endoscope (2) avec une tête d'endoscope (4) et au choix une partie distale de la tête d'endoscope (4), dans lequel la tête d'endoscope (4) est agencée au niveau d'une section d'extrémité distale, pouvant être incurvée de préférence activement d'une tige d'endoscope (3) et la tête d'endoscope (4) ou la partie distale de la tête d'endoscope (4) présente ou porte au moins une optique (6) pour l'imagerie, et
un canal de travail (14) qui s'étend longitudinalement d'un seul tenant dans l'endoscope (2) et dont la sortie de canal de travail est agencée ou réalisée dans la tête d'endoscope (4) ou dans la partie distale de la tête d'endoscope (4),
et un mécanisme de pivotement (10) qui est agencé entre la section d'extrémité de tige distale et la tête d'endoscope (4) ou la partie distale de la tête d'endoscope (4) et qui est réalisé afin de maintenir de manière rabattable ou coudable la tête d'endoscope (4) ou la partie distale de la tête d'endoscope (4), pour laquelle le mécanisme de pivotement (10) présente au moins un segment (16) qui réalise un disque ou une plaque sensiblement ronde selon une vue de dessus et sensiblement en forme de coin selon une vue de côté, disque ou plaque qui présente selon une vue de côté une section périphérique (18) plate et qui s'épaissit en forme de coin en direction de la section périphérique diamétralement opposée afin d'obtenir au niveau de cette section périphérique opposée un maximum d'épaisseur de plaque qui s'étend sur une section périphérique partielle du segment de plaque (16), selon quoi il résulte une surface de contact ou d'appui de segment (22) qui est orientée sensiblement perpendiculairement à l'axe médian de plaque et dans lequel selon une vue de dessus, le canal de travail est réalisé de manière décentralisée au moins partiellement dans cette section périphérique partielle avec une épaisseur de plaque maximale.

2. Endoscope (2) selon la revendication **1,caractérisé en ce que** le mécanisme de pivotement (10) présente une pluralité de segments (16) axialement successifs et réglables de manière angulaire l'un contre l'autre activement au moyen d'au moins un élément d'actionnement, segments qui sont réalisés comme les plaques en forme de coin avec des côtés frontaux orientés l'un par rapport à l'autre en forme de coin, selon quoi chaque plaque reçoit une section d'enveloppe de cylindre avec une longueur axiale minimale (18) et une section d'enveloppe de cylindre opposée avec une longueur axiale maximale (20), dans lequel respectivement deux segments (16) directement contigus sont orientés l'un par rapport à l'autre de telle manière qu'ils s'appuient axialement au niveau de leurs sections d'enveloppe de cylindre respectives de longueur axiale maximale (20) ou reposent l'un sur l'autre, selon quoi un contact de charnière ou d'articulation apparaît au niveau de la surface ou point d'appui ou de repos (22).

3. Endoscope (2) selon la revendication 1, **caractérisé en ce que** la section d'enveloppe de cylindre avec une longueur axiale minimale (18) des segments respectifs (16) présente une longueur axiale inférieure à 2 mm, de préférence inférieure à 1 mm, selon quoi celle-ci réalise une pointe de coin (18) selon une vue de côté.

4. Endoscope (2) selon le préambule de la revendication 1, **caractérisé en ce que** le mécanisme de pivotement (10) présente au moins deux, de préférence trois fils métalliques, câbles Bowden ou barres (40) souples en flexion, rigides aux efforts de cisaillement, qui sont logés dans la tige d'endoscope (3) et qui sont articulés ou fixés distalement au niveau de la tête d'endoscope (4) ou à une partie distale de la tête d'endoscope (4) qui est maintenue exclusivement par le biais des fils métalliques, câbles Bowden ou barres (40) au niveau de la tige d'endoscope (3), dans lequel les fils métalliques ou barres (40) sont prévus et réalisés afin qu'ils puissent déplacer exclusivement la tête d'endoscope ou la partie de tête d'endoscope distale (4) par rapport au segment d'endoscope ou à la partie de tête d'endoscope directement contigüe à ceux-ci de manière axiale et proximale, dans laquelle ils sont poussés et repoussés de préférence au choix ainsi qu'indépendamment l'un de l'autre, selon quoi exclusivement la tête d'endoscope ou la partie distale de la tête d'endoscope (4) est déplacée, en particulier est pivotée par rapport à la tige.

5. Endoscope (2) selon l'une quelconque des revendications 1 à 4, **caractérisé en ce que** l'endoscope (2) présente, outre le mécanisme de pivotement (10), un dispositif d'ajustement fin (52) réalisé séparément de celui-ci pour permettre une orientation plus précise de la sortie de canal de travail.

6. Endoscope (2) selon la revendication 5, **caractérisé en ce que** le dispositif d'ajustement fin (52) est un organe de réglage (70) actionné de manière hydraulique qui peut être alimenté par le biais d'un tuyau (68) en une pression afin de provoquer une inclinaison de la tête d'endoscope (4).

7. Endoscope (2) selon la revendication 6, **caractérisé en ce que** le dispositif d'ajustement fin (52) est précontraint au moyen d'un élément de ressort (56) en particulier magnétisable dans une position basculée au maximum de moins de 15° par rapport à l'axe longitudinal d'endoscope et peut être transféré au moyen d'une bobine magnétique (58) pouvant être alimentée en courant ou d'un fil de traction (72) pouvant être alimenté en force de traction et fixé au niveau de la tête d'endoscope (4) dans le sens inverse à son sens de précontrainte dans une orientation prograde.

8. Endoscope (2) selon la revendication 7, **caractérisé en ce que** le dispositif d'ajustement fin (52) présente au moins deux bagues coniques (62, 64) glissant et reposant l'une contre l'autre et rotatives de manière concentrique l'une par rapport à l'autre.

9. Endoscope (2) selon la revendication 6, **caractérisé en ce que** l'ajustement fin (52) présente un convertisseur à flexion piézoélectrique (74) qui peut être alimenté en une tension par le biais d'une conduite électrique (78).

10. Endoscope (2) selon l'une quelconque des revendications précédentes, **caractérisé en ce qu'**il est un gastro-duodénoscope combiné du type appareil jetable.
